(19) Europäisches Patentamt
European Patent Office
Office européen des brevets

(11) EP 0 723 959 A1

(12) DEMANDE DE BREVET EUROPEEN

(43) Date de publication:
**31.07.1996 Bulletin 1996/31**

(51) Int Cl.[6]: **C07D 211/76**, C07D 453/02, A61K 31/445

(21) Numéro de dépôt: **96400202.6**

(22) Date de dépôt: **29.01.1996**

(84) Etats contractants désignés:
**AT BE CH DE DK ES FR GB GR IE IT LI LU MC NL PT SE**

(30) Priorité: **30.01.1995 FR 9501015**

(71) Demandeur: **SANOFI**
**F-75008 Paris (FR)**

(72) Inventeurs:
- **Emonds-Alt, Xavier**
**F-34980 Combaillaux (FR)**
- **Grossriether, Isabelle**
**F-30700 Uzes (FR)**
- **Proietto, Vincenzo**
**F-34680 St Georges d'Orques (FR)**
- **Van Broeck, Didier**
**F-34570 Murviel Les Montpellier (FR)**

(74) Mandataire: **Gillard, Marie-Louise et al**
**Cabinet Beau de Loménie**
**158, rue de l'Université**
**F-75340 Paris Cédex 07 (FR)**

(54) **Composés hétérocycliques comme antagonistes de récepteurs de la tachykinine, procédé pour leur préparation et compositions pharmaceutiques en contenant**

(57) L'invention concerne des composés de formule :

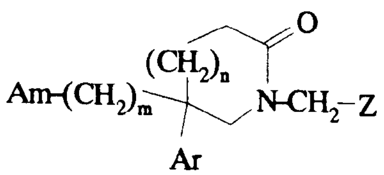

(I)

dans laquelle :

- m est 2 ou 3 ;
- n est 0, 1 ou 2 ;
- Am représente un groupe choisi parmi:

a)

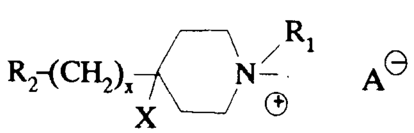

b)

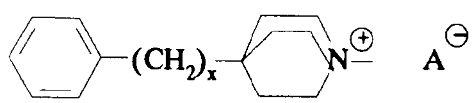

et lorsque n = 0, Am peut également représenter le groupe :

c)

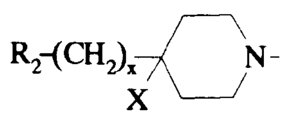

- $R_1$ représente un ($C_1$-$C_4$)alkyle ou un benzyle ;
- $R_2$ représente un phényle substitué ou non ;
- X représente un atome d'hydrogène ou un groupe hydroxyle ;
- x est zéro ou un;
- Ar représente un phényle substitué ou non ; un naphtyle ou un indolyle;
- Z représente un phényle substitué ou non;
- A⊖ représente un anion et ses sels éventuels avec des acides minéraux ou organiques et ses solvates éventuels.

Ces composés ont une forte affinité pour le récepteur $NK_1$ et sont utiles pour la préparation de médicaments pour le traitement de pathologies substance P-dépendantes.

EP 0 723 959 A1

Printed by Jouve, 75001 PARIS (FR)

## Description

La présente invention a pour objet de nouveaux composés hétérocycliques, un procédé pour leur préparation et les compositions pharmaceutiques en contenant en tant que principe actif.

Plus particulièrement, la présente invention concerne une nouvelle classe de composés aromatiques à usage thérapeutique, dans les phénomènes pathologiques qui impliquent le système des tachykinines comme par exemple de manière non limitative et exclusive : la douleur (D. Regoli *et al.,* Life Sciences, 1987, *40*, 109-117),l'allergie et l'inflammation (J.E. Morlay *et al.,* Life Sciences, 1987, *41*, 527-544), l'insuffisance circulatoire (J. Losay *et al.,* 1977, Substance P, Von Euler, I.S. and Pernow ed., 287-293, Raven Press, New York), les troubles gastro-intestinaux (D. Regoli *et al.,* Trends Pharmacol. Sci., 1985, *6*, 481-484), les troubles respiratoires (J. Mizrahi *et al.,* Pharmacology, 1982, 25, 39-50) les troubles neurologiques, les troubles neuropsychiatriques.

Dans les années récentes de nombreux travaux de recherche ont été effectués sur les tachykinines et leurs récepteurs. Les tachykinines sont distribuées à la fois dans le système nerveux central et dans le système nerveux périphérique. Les récepteurs aux tachykinines ont été reconnus et sont classés en trois types : $NK_1$, $NK_2$, $NK_3$. La substance P (SP) est le ligand endogène des récepteurs $NK_1$, la neurokinine A ($NK_A$) celui des récepteurs $NK_2$ et la neurokinine B ($NK_B$), celui des récepteurs $NK_3$.

Les récepteurs $NK_1$, $NK_2$, $NK_3$ ont été mis en évidence chez différentes espèces. Une revue de C.A. Maggi *et al.* fait le point sur les récepteurs aux tachykinines et leurs antagonistes et expose les études pharmacologiques et les applications en thérapeutique humaine (J. Autonomic Pharmacol., 1993, *13*, 23-93).

Parmi les antagonistes spécifiques du récepteur $NK_1$ on peut citer les composés non peptidiques suivants : CP-96345 (J. Med. Chem., 1992, *35*, 2591-2600), RP-68651 (Proc. Natl. Acad. Sci. USA, 1991, *88*, 10208-10212), SR 140333 (Curr. J. Pharmacol., *1993, 250,* 403-413).

De nombreux brevets ou demandes de brevets décrivent des composés actifs sur les récepteurs de tachykinines. Ainsi la demande de brevet européenne 0 512 901 concerne des composés de formule :

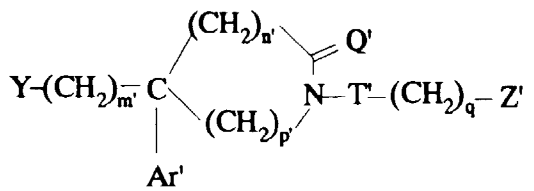

1

dans laquelle notamment :

- Q' représente un atome d'oxygène ou deux atomes d'hydrogène,
- T' = -C(O)- ou $-CH_2-$, et
- Y peut représenter un groupe

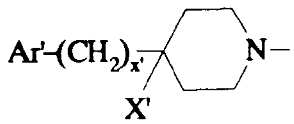

dans lequel

- Ar' représente un phényle éventuellement substitué,
- x'= 0 ou 1 et
- X' représente l'hydrogène, un groupe hydroxyle ou un autre radical monovalent, ainsi que les sels d'ammonium quaternaire avec l'azote de la pipéridine.

Cette demande de brevet ne décrit aucun composé de formule 1 dans laquelle à la fois: Q' est l'oxygène et Y représente un groupe 4-phénylpipéridine ou 4-benzylpipéridine qui soit sous forme de sel d'ammonium quaternaire avec l'azote de la pipéridine, ni aucun composé de formule 1 dans laquelle à la fois : Q' est l'oxygène, n'=p'=1, Y représente un groupe 4-phénylpipéridine ou 4-benzylpipéridine ou 4-hydroxy-4-phénylpipéridine ou 4-hydroxy-4-benzylpipéridine et $-T-(CH_2)_q-Z'$ représente un benzyle substitué.

La demande de brevet européenne 0 591 040 concerne des composés de formule :

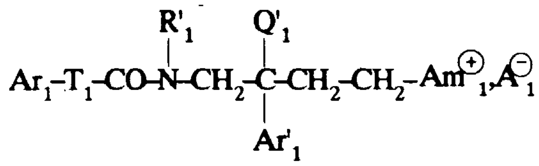

2

dans laquelle notamment :

- $Ar_1$ représente un groupe aromatique ou hétéroaromatique mono-, di- ou tricyclique éventuellement substitué;
- $T_1$ représente une liaison directe, un groupe hydroxyméthylène, alcoxyméthylène ou alkylène ;
- $Ar'_1$ représente un phényle non substitué ou substitué ; un thiényle ; un benzothiényle; un naphtyle ou un indolyle ;
- $Am^{\oplus}_1$ représente le radical

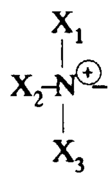

dans lequel $X_1$, $X_2$, $X_3$ ensemble avec l'atome d'azote auquel ils sont liés, forment un système azabicyclique ou azatricyclique, par exemple le groupe 4-phényl-1-azoniabicyclo[2.2.2]octane;

- $R'_1$ a différentes significations ou représente l'hydrogène ;
- $Q'_1$ représente l'hydrogène ou
- $Q'_1$ et $R'_1$ ensemble forment un groupe 1,2-éthylène, 1,3-propylène ou 1,4-butylène.

On a maintenant trouvé des composés non peptidiques qui présentent une très forte affinité pour le récepteur $NK_1$ et une grande spécificité pour ledit récepteur. Ces composés peuvent être utilisés pour la préparation de médicaments utiles dans le traitement de toute pathologie substance P-dépendante.

Par très forte affinité pour le récepteur $NK_1$ humain on entend une affinité caractérisée par une constante d'inhibition Ki inférieure à $5.10^{-9}$M.

Dans les études de fixation d'un ligand, la constante d'inhibition Ki est définie par la relation de Cheng-Prusoff (in Receptor Binding in Drug Research, eds. R.A. O'Brien, Marcel Dekker, New York, 1986) :

$$Ki = \frac{IC_{50}}{1 + \frac{[L]}{Kd}}$$

[L] : concentration du ligand,
Kd : constante de dissociation du ligand,
$IC_{50}$ : concentration qui inhibe 50 % de la fixation du ligand.

Par grande spécificité pour le récepteur $NK_1$ humain, on entend que la constante d'inhibition (Ki) pour le récepteur $NK_1$ humain est au moins 100 fois inférieure à la constante d'inhibition (Ki) pour le récepteur $NK_2$ ou à celle pour le récepteur $NK_3$.

Ainsi selon un de ses aspects, la présente invention concerne des composés de formule :

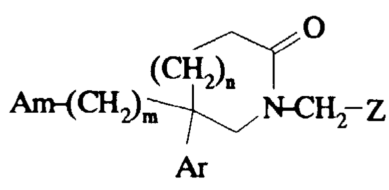

(I)

dans laquelle

- m est deux ou trois ;
- n est 0, 1 ou 2 ;
- Am représente un groupe choisi parmi :

a)

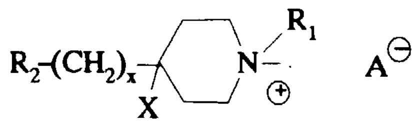

b)

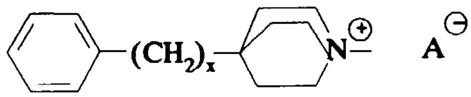

et lorsque n = 0, Am peut également représenter le groupe :

c)

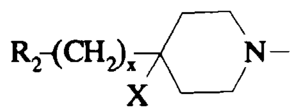

- $R_1$ représente un $(C_1\text{-}C_4)$alkyle ou un benzyle ;
- $R_2$ représente un phényle non substitué ou substitué une ou plusieurs fois par un atome d'halogène, un hydroxyle, un $(C_1\text{-}C_4)$alcoxy, un $(C_1\text{-}C_4)$alkyle, un trifluorométhyle, lesdits substituants étant identiques ou différents ;
- X représente un atome d'hydrogène ou un groupe hydroxyle ;
- x est zéro ou un ;
- Ar représente un phényle non substitué ou substitué une ou plusieurs fois par un atome d'halogène ; un naphtyle ou un indolyle ;
- Z représente un phényle substitué une ou plusieurs fois par un atome d'halogène, un $(C_1\text{-}C_4)$ alkyle, un trifluorométhyle ;
- $A^{\ominus}$ représente un anion ;

ainsi que leurs sels éventuels avec des acides minéraux ou organiques et leurs solvates éventuels.

Les composés de formule (I) selon l'invention comprennent aussi bien les isomères optiquement purs que les racémiques ainsi que les isomères axiaux et équatoriaux lorsque Am a la valeur a).

Lorsque Am représente c), on peut former des sels des composés de formule (I). Ces sels comprennent aussi bien ceux des acides minéraux ou organiques qui permettent une séparation ou une cristallisation convenable des composés de formule (I), tels que l'acide picrique oxalique ou un acide optiquement actif, par exemple un acide mandélique ou camphosulfonique, que ceux qui forment des sels pharmaceutiquement acceptables tels que le chlorhydrate, le bromhydrate, le sulfate, l'hydrogénosulfate, le dihydrogénophosphate, le méthanesulfonate, le méthylsulfate, le maléate, le fumarate, le 2-naphtalènesulfonate, le benzènesulfonate, le gluconate, le citrate, l'iséthionate, le *p*-toluènesulfonate.

Les anions sont ceux normalement utilisés pour salifier les ions d'ammonium quaternaires, de préférence les ions chlorure, bromure, iodure, acétate, hydrogénosulfate, méthanesulfonate, paratoluènesulfonate, benzènesulfonate.

Préférentiellement, on utilise les anions pharmaceutiquement acceptables par exemple le chlorure, le méthanesulfonate, le paratoluènesulfonate, le benzènesulfonate.

Dans la présente description les groupes alkyles ou les groupes alcoxy sont droits ou ramifiés ; par atome d'halogène on entend un atome de chlore, de brome, de fluor ou d'iode.

Avantageusement, la présente invention concerne les composés de formule (I) dans laquelle m = 2.

Les composés de formule (I) dans lesquels Z est un groupe choisi parmi diméthylphényle, trifluorométhylphényle et bistrifluorométhylphényle sont des composés préférés selon l'invention.

Selon la présente invention, on préfère les composés de formule (I) dans laquelle

- Ar représente un 3,4-dichlorophényle ou un 3,4-difluorophényle ;
- Z représente un 3,5-diméthylphényle, un 3,5-bis(trifluorométhyl)phényle ou un 2,4-bis(trifluorométhyl) phényle ;
- et soit n = 0, 1 ou 2 et Am représente a) ou b), - soit n = 0 et Am représente c) ;

- $A^{\ominus}$ représente un anion pharmaceutiquement acceptable.

Selon la présente invention, on préfère également les composés de formule :

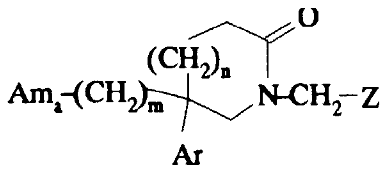

(Ia)

dans laquelle $Am_a$ représente le groupe b) tel que défini pour (I) et n, m, Ar et Z sont tels que définis pour (I) et leurs solvates éventuels.

Tout particulièrement, on préfère les sels pharmaceutiquement acceptables des composés de formule :

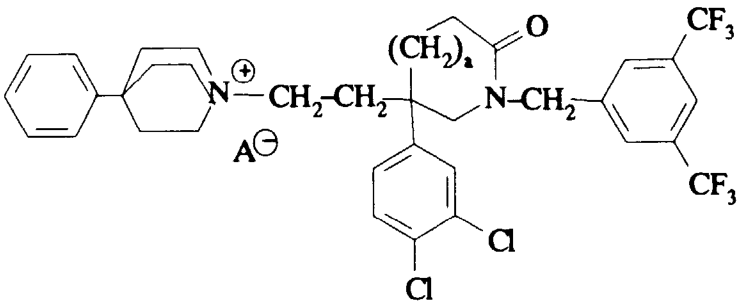

(I')

dans laquelle a est 0 ou 1 ;
et leurs solvates éventuels.

Selon un autre de ses aspects, la présente invention concerne un procédé pour la préparation des composés de formule (I) caractérisé en ce que :

1) on traite un composé de formule :

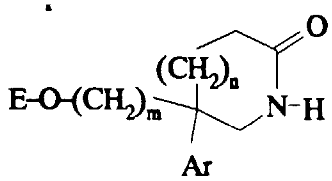

(II)

dans laquelle m, n et Ar sont tels que définis pour (I) et E représente un groupe O-protecteur tel que le tétrahydropyran-2-yle, le benzoyle ou un acyle en $C_1$-$C_4$ ;
avec un dérivé halogéné de formule :

$$Hal\text{-}CH_2\text{-}Z \qquad (III)$$

dans laquelle Z est tel que défini pour (I) et Hal représente un atome d'halogène, préférentiellement le brome ou le chlore ;
2) on transforme le groupe O-protecteur en groupe hydroxyle par action d'un acide ou d'une base ;
3) on traite l'alcool ainsi obtenu de formule :

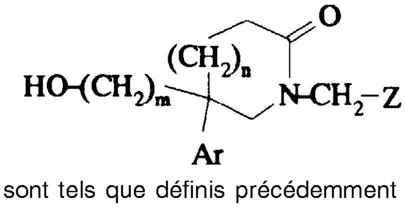

(IV)

dans laquelle Ar, Z, m et n sont tels que définis précédemment avec le chlorure de méthanesulfonyle, le chlorure de benzènesulfonyle, le chlorure de paratoluènesulfonyle ou le chlorure de trifluorométhanesulfonyle;
4) on fait réagir le composé ainsi obtenu de formule :

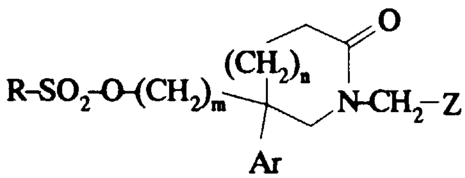

(V)

dans laquelle R représente un groupe méthyle, phényle, paratolyle ou trifluorométhyle ;
avec une amine secondaire ou tertiaire AmH ou Am dans un solvant organique, à une température comprise entre la température ambiante et 120°C ;
5) on isole le produit ainsi obtenu et, éventuellement, on échange l'anion sulfonate avec un autre anion, et, éventuellement, on transforme le produit ainsi obtenu en un de ses sels.

A l'étape 2, la déprotection est effectuée en milieu acide, par exemple en présence d'acide chlorhydrique lorsque E représente un tétrahydropyran-2-yle ou en milieu basique, par exemple en présence de soude ou d'hydroxyde de lithium lorsque E représente un benzoyle ou un acyle en $C_1$-$C_4$.

A la dernière étape l'anion sulfonate peut être échangé, *in situ* ou après isolement du composé de formule (I) dans laquelle $A^{\ominus}$ est l'ion sulfonate, par un autre anion $A^{\ominus}$, selon les méthodes conventionnelles, par exemple par échange en solution avec une solution saturée de chlorure de sodium ou, avec une solution d'acide chlorhydrique lorsque $A^{\ominus}$ représente un anion chlorure, ou par échange de l'anion par élution du composé (I) sur une résine échangeuse d'ion, par exemple l'Amberlite IRA68 ou Duolite A375.

Lorsque Am représente un groupe a), les sels d'ammonium quaternaires formés avec l'azote de la pipéridine sont préparés par réaction des bases libres des composés de formule (I) avec un excès d'agent alkylant de formule : A-$R_1$ dans lequel A est tel que défini précédemment pour (I), de préférence un chlorure ou un iodure et $R_1$ est tel que défini précédemment pour (I) et on chauffe le mélange réactionnel dans un solvant, par exemple le dichlorométhane, le chloroforme, l'acétone ou l'acétonitrile, à une température comprise entre la température ambiante et le reflux, pendant une à plusieurs heures, pour obtenir après traitement selon les méthodes habituelles un mélange des isomères axiaux et équatoriaux des sels d'ammonium quaternaires. De préférence, $A^{\ominus}$ représente un iodure qui peut être échangé par un autre anion ou par un anion pharmaceutiquement acceptable, par exemple par élution du composé (I) sur une résine échangeuse d'ion, telle l'Amberlite IRA68 ou Duolite A375. Les isomères sont séparés selon les méthodes habituelles, par exemple par chromatographie ou par recristallisation.

La résolution des mélanges racémiques de composés (I) ou d'intermédiaires tels que (IV) permet d'isoler les énantiomères en utilisant des méthodes connues de l'homme de l'art.

La préparation des composés de formule (II) est effectuée selon les procédés décrits dans EP-A-0 512 901.

Les dérivés halogénés de formule (III) sont connus ou préparés par des méthodes connues.

La 4-phénylpipéridine, la 4-benzylpipéridine, la 4-hydroxy-4-phénylpipéridine et la 4-hydroxy-4-benzylpipéridine sont commerciales.

La 4-phényl-1-wabicyclo[2.2.2]octane, ou 4-phénylquinuclidine et la 4-benzyl-1-azabicyclo[2.2.2]octane ou 4-benzylquinuclidine sont préparées selon T. Perrine, J. Org. Chem., 1957, 22, 1484-1489.

Les composés de formule (I) ci-dessus comprennent également ceux dans lesquels un ou plusieurs atomes d'hydrogène, de carbone, de fluor ou d'iode ont été remplacés par leur isotope radioactif par exemple le tritium, le carbone-14, le fluor-19 ou l'iode 125. De tels composés marqués sont utiles dans des travaux de recherche, de métabolisme, de pharmacocinétique ou dans des essais biochimiques en tant que ligands de récepteurs et peuvent être utilisés comme outils pharmacologiques chez l'homme ou chez l'animal.

Les composés selon l'invention ont fait l'objet d'essais biochimiques.

L'affinité des composés de formule (I) pour les récepteurs aux tachykinines a été évaluée in vitro par plusieurs essais biochimiques utilisant des radioligands :

1°) La liaison de [$^{125}$I]BH-SP (Substance P marquée à l'iode 125 à l'aide du réactif de Bolton-Hunter) aux récepteurs $NK_1$ des cellules lymphoblastiques humaines.
2°) La liaison [$^{125}$I]His-$NK_A$ aux récepteurs $NK_2$ du duodénum ou de la vessie de rat.
3°) La liaison [$^{125}$I]His[MePhe$^7$]$NK_B$ aux récepteurs $NK_3$ du cortex cérébral de rat, du cortex cérébral de cobaye et du cortex cérébral de gerbille ainsi qu'aux récepteurs clones $NK_3$ humains exprimés par des cellules CHO (Buell et al., FEBS Letters, 1992, 299, 90-95).

Les essais ont été effectués selon X. Emonds-Alt et al. (Eur. J. Pharmacol., 1993, 250, 403-413).

Les composés selon l'invention inhibent fortement la liaison aux récepteurs $NK_1$ des cellules lymphoblastiques humaines IM9. la constante d'inhibition Ki pour les récepteurs des cellules lymphoblastiques est inférieure à

$5.10^{-9}$M. Pour les mêmes composés on a constaté que la constante d'inhibition (Ki) pour les récepteurs $NK_3$ clonés humains est supérieure ou égale à $10^{-7}$M et que la constante d'inhibition (Ki) pour le récepteur $NK_2$ du duodénum ou de la vessie de rat est supérieure ou égale à $10^{-7}$M.

L'activité des composés selon la présente invention comme inhibiteurs de la liaison aux récepteurs $NK_1$ est nettement supérieure à celle de composés voisins décrits dans l'art antérieur.

Ainsi, l'isomère axial de l'iodure de 1-méthyl-1-[2-[1-[3,5-bis(trifluorométhyl) benzyl]-5-(3,4-dichlorophényl)-2-oxopipérid-5-yl]éthyl]-4-phénylpipéridinium présente une constante d'inhibition Ki de la substance P au récepteur lymphoblastique humain de l'ordre de $0,1.10^{-9}$M alors que le chlorhydrate de 5-[2-(4-benzyl-1-pipérid-1-yl)éthyl]-5-(3,4-dichlorophényl)-1-benzylpipérid-2-one décrit dans la demande EP-0 512 901 à l'exemple 1 (composé non substitué sur le benzyle) présente une constante d'inhibition Ki de l'ordre de $10^{-6}$M mesurée dans les mêmes conditions.

De même le chlorhydrate de 4-(3,4-dichlorophényl)-4-[2-(4-hydroxy-4-phénylpipérid-1-yl)éthyl]-1-[3,5-bis(trifluorométhyl)benzyl]pyrrolidin-2-one présente une constante d'inhibition Ki de la substance P au récepteur lymphoblastique humain de l'ordre de $0,1.10^{-9}$M alors que le chlorhydrate de 1-benzyl-4-(3,4-dichlorophényl)-4-[2-(4-hydroxy-4-phénylpipérid-1-yl)éthyl]pipérid-2-one décrit dans la demande EP-0 512 901 à l'exemple 3 présente un Ki de l'ordre de $10^{-6}$M mesuré dans les mêmes conditions.

Les composés de la présente invention sont notamment des principes actifs de compositions pharmaceutiques, dont la toxicité est compatible avec leur utilisation en tant que médicaments.

Les composés de formule (I) ci-dessus peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,5 à 4000 mg par jour, plus particulièrement de 2,5 à 1000 mg par jour selon l'âge du sujet à traiter ou le type de traitement : prophylactique ou curatif.

Pour leur utilisation comme médicaments, les composés de formule (I) sont généralement administrés en unités de dosage. Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I).

Dans les compositions pharmaceutiques de la présente invention pour l'administration par voie orale, sublinguale, inhalée, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les implants, les formes d'administration sous-cutanée, intramusculaire, intraveineuse, intranasale ou intraoculaire et les formes d'administration rectale.

Lorsque l'on prépare une composition solide sous forme de comprimés, on mélange le principe actif principal avec un véhicule pharmaceutique tel que la silice, la gélatine, l'amidon, le lactose, le stéarate de magnésium, le talc, la gomme arabique ou analogues. On peut enrober les comprimés de saccharose de divers polymères, ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant tel qu'un glycol ou un ester de glycérol et en incorporant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Pour une administration par inhalation, on utilise un aérosol contenant par exemple du trioléate de sorbitane ou de l'acide oléique ainsi que du trichlorofluorométhane, du dichlorofluorométhane, du dichlorotétrafluoroéthane ou tout autre gaz propulseur biologiquement compatible ; on peut également utiliser un système comprenant le principe actif, seul ou associé à un excipient, sous forme de poudre.

Le principe actif peut être formulé également sous forme de microcapsules, éventuellement avec un ou plusieurs supports ou additifs.

Dans chaque unité de dosage le principe actif de formule (I) est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,5 à 1000 mg de principe actif, de préférence de 2,5 à 250 mg, devant être administrée une a quatre fois par jour.

Selon un autre de ses aspects, la présente invention concerne l'utilisation des produits de formule (I) pour la préparation de médicaments destinés à traiter des troubles physiologiques associés à un excès de tachykinines, notamment de Substance P et toutes les pathologies Substance-P-dépendantes du système respiratoire, gastro-intestinal, urinaire, immunitaire, cardiovasculaire et du système nerveux central ainsi que la douleur et la migraine.

Par exemple et de manière non limitative :

- douleurs aigües et chroniques liées par exemple à la migraine, aux douleurs du cancéreux et de l'angineux, aux processus inflammatoires chroniques tels que l'ostéoarthrite et l'arthrite rhumatoïde,
- les inflammations telles que les maladies respiratoires chroniques obstructives, l'asthme, les allergies, les rhinites, les toux, les bronchites, l'hypersensibilité par exemple aux pollens et aux acariens, les arthrites rhumatoïdes, les ostéoarthrites, le psoriasis, les colites ulcératives, la maladie de Crohn, l'inflammation des intestins (colon irritable), la prostatite, la vessie neurologique, la cystite, l'urétrite, la néphrite,
- les maladies du système immunitaire liées à la suppression ou à la stimulation des fonctions des cellules immunes par exemple l'arthrite rhumatoïde, le psoriasis, la maladie de Crohn, la diabète, le lupus,
- les maladies du système nerveux central du type neuropsychiatrique ou neurologique telles que l'anxiété, la dépression, la psychose, la schizophrénie, la manie, la démence, l'épilepsie, la maladie de Parkinson, la maladie d'Alzheimer, la drogue-dépendance, le syndrome de Down et la chorée d'Huntington ainsi que les maladies neurodégénératives,
- les maladies du système gastro-intestinal telles que nausées, vomissement, colon irritable, ulcères gastriques et duodénaux, diarrhées, hypersécrétions,
- les maladies du système cardiovasculaire telles que les l'hypertension, les aspects vasculaires de la migraine, oedèmes, thrombose, l'angine de poitrine et les spasmes vasculaires,
- les troubles de la fréquence et du rythme cardiaques, en particulier ceux qui sont occasionnés par la douleur ou le stress.

La présente invention inclut aussi une méthode pour traiter lesdites affections aux doses indiquées ci-dessus.

Dans les Préparations et dans les exemples on utilise les abréviations suivantes :

EtOH : éthanol
MeOH : méthanol
Ether : éther diéthylique
Ether iso : éther diisopropylique
DMF : diméthylformamide
AcOEt : acétate d'éthyle
DCM : dichlorométhane
THF: tétrahydrofurane
NaOH : soude
$KHCO_3$ : hydrogénocarbonate de potassium
$NaHCO_3$ : hydrogénocarbonate de sodium
NaCl : chlorure de sodium
Triton B : solution à 40 % d'hydroxyde de N-benzyltriméthylammonium dans le MeOH.
$Na_2SO_4$ : sulfate de sodium
$MgSO_4$ : sulfate de magnésium
iPr: isopropyle
TA : température ambiante
F : point de fusion
RMN : résonnance magnétique nucléaire.
s : singulet
sd : singulet dédoublé
d : doublet
sept : septuplet
m : massif
mult : multiplet

PREPARATION 1

5-(3,4-Dichlorophényl)-5-[2-(tétrahydropyran-2-yloxy)éthyl]pipérid-2-one.

A) 2-(3,4-Dichlorophényl)-4-(tétrahydropyran-2-yloxy)butanenitrile.

20 g d'hydrure de sodium à 55-60 % dans l'huile sont mis en suspension dans 200 ml de tétrahydrofurane sec. On ajoute goutte à goutte à 20°C, en 30 minutes, une solution de 85 g de 3,4-dichlorophénylacétonitrile dans 500 ml de THF puis on agite le mélange réactionnel à TA pendant 2 heures. Le mélange est refroidi à -20°C et on ajoute une solution de 98 g de 2-(2-bromoéthoxy)tétrahydropyrane dans 100 ml de THF, on laisse revenir le mélange à TA et après 2 heures on ajoute une solution de 50 g de chlorure d'ammonium dans 3 litres d'eau. On extrait avec 1,5 litres d'éther, lave avec une solution saturée de chlorure de sodium, décante, sèche sur $MgSO_4$ et concentre sous vide.

Le résidu est chromatographié sur gel de silice, éluant : dichlorométhane. Les fractions de produit pur sont concentrées sous vide pour fournir 83,6 g d'une huile.

B) 4-Cyano-4-(3,4-dichlorophényl)-6-(tétrahydropyran-2-yloxy)hexanoate d'éthyle.

21 g du nitrile préparé précédemment selon A) sont mis en solution dans 100 ml de THF, puis on ajoute goutte à goutte et à température ambiante une solution de 0,067 mole de diisopropylamidure de lithium dans 100 ml de THF et agite le mélange réactionnel pendant une heure à TA. On ajoute alors 12 g de 3-bromopropionate d'éthyle et chauffe à 50°C pendant deux heures. Le mélange est refroidi on le verse dans une solution saturée de chlorure d'ammonium et extrait à l'éther, puis on lave à l'eau, sépare la phase éthérée par décantation, la sèche sur $Na_2SO_4$ et concentre sous vide. Le résidu est purifié par chromatographie sur gel de silice, éluant : dichlorométhane/acétate d'éthyle 100/1 (v/v). La concentration des fractions pures fournit 13 g du composé attendu.

C) 5-(3,4-Dichlorophényl)-5-[2-(tétrahydropyran-2-yloxy)éthyl]pipérid-2-one.

13 g du composé préparé précédemment sont mis en solution dans 250 ml d'éthanol et 40 ml d'ammoniaque et sont hydrogénés à température ambiante et pression atmosphérique en présence de nickel de Raney®. Lorsque le volume théorique d'hydrogène est absorbé, on filtre le mélange sur Célite® et on concentre le filtrat sous vide. Le résidu est repris dans l'eau, extrait à l'éther, puis on lave la phase éthérée à l'eau, la sèche sur $MgSO_4$ et concentre sous vide pour obtenir 9 g du produit attendu.

PREPARATION 2.

5-[2-(Tétrahydropyran-2-yloxy)éthyl]-5-(3,4-difluorophényl)pipérid-2-one.

A) 2-(3,4-Difluorophényl)-4-(tétrahydropyran-2-yloxy)butanenitrile.

14,4 g d'hydrure de sodium à 60 % dans l'huile sont mis en suspension dans 250 ml de THF et refroidis dans un bain d'eau. On ajoute goutte à goutte une solution de 50 g de 3,4-difluorophénylacétonitrile dans 50 ml de THF puis on laisse 3 heures à TA et on ajoute goutte à goutte une solution de 68,16 g de 2-(2-bromoéthoxy)tétrahydropyrane dans 100 ml de THF. Après une nuit à TA, on acidifie avec un tampon pH2, évapore le THF, reprend par de l'eau et extrait à l'éther. On lave 2 fois avec une solution tampon pH4, puis on lave à l'eau et par une solution saturée de chlorure de sodium. On décante, sèche la phase organique sur $MgSO_4$ et concentre sous vide. Le résidu est chromatographié sur silice en éluant par du toluène pur puis contenant jusqu'à 3 % d'AcOEt. On obtient 49,5 g du produit attendu.

B) 6-(Tétrahydropyran-2-yloxy)-4-(3,4-difluorophényl)-4-cyanohexanoate de méthyle.

Le produit obtenu à l'étape précédente (28,12 g) est placé dans 100 ml de THF, on ajoute 1 ml de Triton B et on chauffe à reflux puis on ajoute goutte à goutte 9,46 g d'acrylate de méthyle. On laisse revenir à TA, évapore le solvant, reprend par de l'eau et extrait à l'éther. On lave 2 fois par de l'eau, 2 fois par une solution tampon à pH4, par de l'eau puis par une solution saturée de chlorure de sodium. On décante, sèche la phase organique sur $MgSO_4$ et concentre sous vide pour obtenir 34,6 g du produit attendu.

C)5-[2-(Tétrahydropyran-2-yloxy)éthyl]-5-(3,4-difluorophényl)pipérid-2-one.

Le produit (34,6 g) obtenu à l'étape précédente est mis en solution dans 500 ml d'EtOH 95. On ajoute 5 g de $KHCO_3$ solide puis du nickel de Raney®. On hydrogène à 60°C sous 20,4 bars. Après 4 heures, on filtre sur Célite® et évapore le solvant. On reprend par de l'eau, extrait par AcOEt, lave 2 fois à l'eau et par une solution saturée de chlorure de sodium. On sèche la phase organique sur $MgSO_4$ et concentre sous vide. On obtient 32 g du produit attendu.

PREPARATION 3.

6-(3,4-Dichlorophényl)-6-[2-(tétrahydropyran-2-yloxy)éthyl]perhydroazepin-2-one.

A) 5-Cyano-5-(3,4-dichlorophenyl)pentanoate d'éthyle.

5,9 g d'hydrure de sodium à 55 % dans l'huile sont mis en suspension dans 50 ml de THF ; on refroidit dans un bain de glace et on ajoute goutte à goutte 25 g de 3,4-dichlorophénylacétonitrile dans 25 ml de THF; on refroidit à nouveau dans un bain de glace et on ajoute goutte à goutte 26,21 g de 4-bromobutanoate d'éthyle en solution dans 25 ml de THF. Après une nuit à TA, on évapore à sec puis on reprend par une solution d'acide chlorhydrique 2N et extrait à l'éther. On lave par une solution d'acide chlorhydrique 2N puis par une solution saturée de chlorure de sodium. On sèche la phase organique sur $MgSO_4$ puis concentre sous vide et on chromatographie le résidu sur silice en éluant par du toluène pur jusqu'à un mélange toluène/AcOEt (100/3 ; v/v). On obtient 17 g du produit attendu.

B) 5-Cyano-5-(3,4-dichlorophényl)-7-(tétrahydropyran-2-yloxy)heptanoate d'éthyle.

2,5 g d'hydrure de sodium à 55 % dans l'huile sont mis en suspension dans 50 ml de DMF, on refroidit à -20°C et on ajoute goutte à goutte un mélange de 11,8 g de 2-(2-bromoéthoxy)tétrahydropyrane et 17 g du composé obtenu à l'étape précédente dans 50 ml de DMF. Après une nuit à TA, on évapore à sec, reprend par une solution tampon pH4 puis extrait par AcOEt. On lave avec une solution tampon pH4, lave à l'eau puis par une solution saturée de chlorure de sodium. La phase organique est séchée sur $MgSO_4$ puis concentrée et le résidu est chromatographié sur silice en éluant par du toluène pur jusqu'à un mélange toluène/AcOEt (100/10 ; v/v). On obtient 15,4 g du produit attendu.

C) 5-(Aminométhyl)-5-(3,4-dichlorophényl)-7-(tétrahydropyran-2-yloxy) heptanoate d'éthyle.

Le produit obtenu à l'étape précédente (15,4 g) est mis en solution dans 200 ml de MeOH, on ajoute 25 ml d'une solution saturée d'ammoniac dans le MeOH puis on hydrogène à 40°C sous pression atmosphérique en présence de nickel de Raney®. On filtre le catalyseur, évapore à sec et reprend le résidu par de l'éther. On lave à l'eau, par une solution saturée de chlorure de sodium, sèche sur $MgSO_4$ et concentre sous vide pour obtenir 12,8 g du produit attendu.

D) 6-(3,4-Dichlorophényl)-6-[2-(tétrahydropyran-2-yloxy)éthyl]perhydroazepin-2-one.

Le produit obtenu à l'étape précédente (12,8 g) est mis en solution dans 200 ml de xylène et chauffé à reflux pendant 48 heures. On évapore à sec puis on chromatographie le résidu sur silice en éluant par DCM pur jusqu'à un mélange DCM/MeOH (98/2 ; v/v). On obtient 10,1 g du produit attendu.

PREPARATION 4

4-(3,4-Dichlorophényl)-4-[2-(tétrahydropyran-2-yloxy)èthyl]pyrrolidin-2-one.

A) 2-(3,4-Dichlorophényl)-4-(tétrahydropyran-2-yloxy)butanenitrile.

Ce composé est préparé à la Préparation 1, étape A.

B) 3-Cyano-3-(3,4-dichlorophényl)-5-(tétrahydropyran-2-yloxy)pentanoate d'éthyle.

57 g du composé de l'étape A sont mis en solution dans 150 ml de DMF. On ajoute par petites fractions 7,85 g d'hydrure de sodium à 55 % dans l'huile. Après 1 heure de chauffage à 60°C, on refroidit à TA et ajoute goutte à goutte 30,8 g de bromoacétate d'éthyle dans 50 ml de DMF. Après 2 heures sous agitation à TA, on évapore le DMF sous pression réduite, on extrait à l'éther puis lave à l'eau jusqu'à pH neutre. Après séchage sur $MgSO_4$ et évaporation des solvants, le résidu est chromatographié sur silice en éluant par un mélange DCM/AcOEt (95/5 ; v/v). On obtient 29 g

du composé attendu.

C)4-(3,4-Dichlorophényl)-4-[2-(tétrahydropyran-2-yloxy)éthyl]pyrrolidin-2-one.

On hydrogène à pression atmosphérique et TA 8 g du composé obtenu à l'étape B en solution dans 100 ml d'EtOH 100, 5 ml d'eau et 0,6 g de $NaHCO_3$. On filtre le catalyseur et évapore sous vide le filtrat. On extrait le résidu au DCM, lave à l'eau, sèche sur $MgSO_4$ et évapore sous vide le solvant. On obtient 6 g du produit attendu.

PREPARATION 4 BIS

4-(3,4-Dichlorophényl)-4-[2-(tétrahydropyran-2-yloxy)éthyl]pyrrolidin-2-one.

A) 3-Cyano-3-(3,4-dichlorophényl)-1,5-pentanedioate de diéthyle.

A 10 g de 3,4-dichlorophénylacétonitrile en solution dans 50 ml de THF, on ajoute 4,3 g d'hydrure de sodium à 60 % dans l'huile et on laisse sous agitation jusqu'à la fin du dégagement d'hydrogène. On refroidit à 0°C et ajoute goutte à goutte 18 g de bromoacétate d'éthyle en solution dans 50 ml de THF. On laisse revenir à TA et laisse une nuit sous agitation. On évapore à sec, reprend à l'éther, lave avec une solution tampon à pH = 2 et sèche sur $MgSO_4$. On chromatographie le résidu sur silice en éluant par un mélange DCM/AcOEt (80/20 ; v/v). On obtient 15 g du composé attendu.

B) 4-(3,4-dichlorophényl)-4-(éthoxycarbonylméthyl)pyrrolidin-2-one.

15 g du composé obtenu à l'étape précédente sont mis en solution dans 100 ml d'éthanol et hydrogénés à TA et à pression atmosphérique en présence de nickel de Raney®. Quand le volume théorique d'hydrogène à consommer est atteint, on filtre le catalyseur, évapore à sec, reprend à l'éther, lave à l'eau et sèche sur $MgSO_4$ . On obtient 12 g du composé attendu.

C) 4-(3,4-dichlorophényl)-4-(2-hydroxyéthyl)pyrrolidin-2-one.

12 g du produit obtenu à l'étape précédente et 6 g de borohydrure de calcium sont mis en solution dans 50 ml de THF. Après 2 heures sous agitation à 0°C, on laisse revenir à TA puis on verse le milieu réactionnel sur de l'eau et acidifie à pH 1 par addition d'une solution concentrée d'acide chlorhydrique. On extrait à l'acétate d'éthyle, lave à l'eau et sèche sur $MgSO_4$. Le résidu est chromatographié sur silice en éluant par le mélange DCM/MeOH (100/5 ; v/v). On obtient 10 g du composé attendu.

D) 4-(3,4-Dichlorophényl)-4-[2-(tétrahydropyran-2-yloxy)éthyl]pyrrolidin-2-one.

A 10 g du produit obtenu à l'étape précédente, en solution dans 50 ml de DCM avec 0,1 g d'acide paratoluènesulfonique, on ajoute goutte à goutte à 0°C 3 g de 3,4-dihydro-2H-pyrane en solution dans 10 ml de THF. On laisse ensuite une nuit à TA, évapore à sec, reprend par de l'éther, lave à l'eau, par une solution de $NaHCO_3$ saturée et sèche sur $MgSO_4$. Le résidu est chromatographié sur silice en éluant par un mélange DCM/MeOH (100/5 ; v/v). On obtient 12 g du composé attendu.

PREPARATION 5

5-(3,4-Dichlorophényl)-5-[3-(tétrahydropyran-2-yloxy)propyl]pipérid-2-one.

A) 2-(3,4-Dichlorophényl)-5-(tétrahydropyran-2-yloxy)pentanenitrile.

A une solution de 13,35 g de 3,4-dichlorophénylacétonitrile dans 100 ml de THF, on ajoute à TA et par petites fractions 2,8 g d'hydrure de sodium à 60 % dans l'huile et laisse 3 heures sous agitation à TA. On refroidit à -20°C le mélange réactionnel, ajoute goutte à goutte une solution de 16 g de 2-(3-bromopropoxy)tétrahydropyrane dans 30 ml de THF et laisse 3 heures sous agitation en laissant remonter la température à TA. On concentre sous vide le mélange réactionnel, extrait le résidu à l'éther, lave la phase organique à l'eau, par une solution tampon pH = 4, à l'eau, sèche sur $MgSO_4$ et évapore sous vide le solvant. On chromatographie le résidu sur silice en éluant par le mélange toluène/AcOEt (100/3 ; v/v). On obtient 11 g du produit attendu.

B) 4-Cyano-4-(3,4-dichlorophényl)-7-(tétrahydropyran-2-yloxy)heptanoate d'éthyle.

A une solution de 11 g du composé obtenu à l'étape précédente et 1 ml de Triton B dans 25 ml de dioxane on ajoute à TA et goutte à goutte 3,3 g d'acrylate d'éthyle puis laisse 1 heure sous agitation à TA. On dilue le mélange réactionnel par ajout de 200 ml d'éther, lave quatre fois la phase organique à l'eau, par une solution saturée de chlorure de sodium, sèche sur $MgSO_4$ et évapore sous vide le solvant. On obtient 11 g du produit attendu.

C)5-(3,4-Dichlorophényl)-5-[3-(tétrahydropyran-2-yloxy)propyl]pipérid-2-one.

On hydrogène à 40°C et sous 10,2 bars un mélange de 11 g du composé obtenu à l'étape précédente, 1 g de nickel de Raney® et 0,77 g de $NaHCO_3$ dans 120 ml d'EtOH 95. On filtre le catalyseur sur Célite® et concentre sous vide le filtrat. On extrait le résidu au DCM, lave la phase organique à l'eau, par une solution saturée de chlorure de sodium, sèche sur $MgSO_4$ et évapore sous vide le solvant. On chromatographie le résidu sur silice en éluant par le mélange DCM/MeOH (97/3 ; v/v). On obtient 6,8 g du produit attendu.

EXEMPLE 1

Chlorure de 1-[2-[1-[3,5-bis(trifluorométhyl)benzyl]-5-(3,4-dichlorophényl)-2-oxopipérid-5-yl]éthyl]-4-phénylquinuclidinium, monohydrate.

(I) : Am = 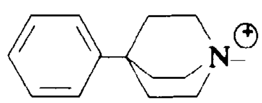 ; Ar = 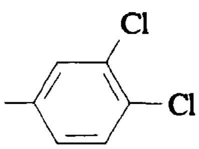 ;

m = 2; n = 1 ;

Z = 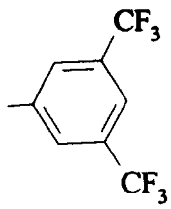 ;

$A^{\ominus} = Cl^{\ominus}$

A) 5-(2-Hydroxéthyl)-5-(3,4-dichorophényl)-1-[3,5-bis(trifluorométhyl) benzyl] pipérid-2-one.

On agite une heure à TA, 1,86 g du composé obtenu à la PRÉPARATION 1 et 0,61 g du *tert*-butylate de potassium dans 30 ml de THF ; on ajoute 1,54 g de bromure de 3,5-bis(trifluorométhyl)benzyle et on chauffe à 50°C pendant 2 heures. On évapore à sec le milieu réactionnel. On reprend par 25 ml de méthanol, refroidit à 0°C, ajoute de l'éther chlorhydrique jusquà pH = 1 puis évapore à sec. On reprend le résidu obtenu par de l'acétate d'éthyle, lave 3 fois par de l'eau, sèche sur $MgSO_4$, évapore sous vide le solvant et l'on obtient 2,6 g du produit attendu.

B) 5-(2-Mésyloxyéthyl)-5-(3,4-dichlorophényl)-1-[3,5-bis(trifluorométhyl) benzyl] pipérid-2-one.

On refroidit à 0°C un mélange du produit obtenu à l'étape précédente (2,6 g) et 0,66 g de triéthylamine dans 30 ml de DCM. Puis on ajoute 0,63 g de chlorure de méthanesulfonyle dans 10 ml de DCM. Après 15 minutes, on évapore à sec puis on extrait à l'éther, lave à l'eau puis par une solution saturée de chlorure de sodium, sèche sur $MgSO_4$, évapore sous vide le solvant et l'on obtient 2,95 g du produit attendu.

C) Chlorure de 1-[2-[1-[3,5-bis(trifluorométhyl)benzyl]-5-(3,4-dichlorophényl)-2-oxopipérid-5-yl]éthyl]-4-phénylquinuclidinium, monohydrate.

On chauffe à 80°C pendant 3 heures un mélange contenant le mésylate obtenu à l'étape précédente (2,95 g) et 1,41 g de 4-phénylquinuclidine dans 1,2 ml de DMF. Après évaporation à sec, on reprend par du DCM puis on lave deux fois par une solution d'acide chlorhydrique 2N puis par de l'eau. On sèche sur $MgSO_4$, concentre sous vide puis

on chromatographie sur silice en éluant par un mélange DCM/MeOH (95/5 ; v/v). On obtient 0,64 g du produit attendu après cristallisation dans le pentane, F =158-160°C.

EXEMPLE 2

Chlorure de 1-[2-[1-[3,5-bis(trifluorométhyl)benzyl]-5-(3,4-difluorophényl)2-oxopipérid-5-yl]éthyl]-4-phénylquinuclidinium, dihydrate.

(I) : Am = 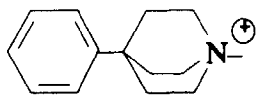 ; Ar = 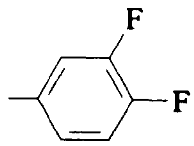 ;

m = 2 ; n = 1;

Z = 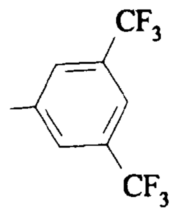 ;

$A^{\ominus} = Cl^{\ominus}$

A) 5-(3,4-Difluorophényl)-5-[2-(tétrahydropyran-2-yloxy)éthyl]-1-[3,5-bis(trifluorométhyl)benzyl]pipérid-2-one.

5 g du composé obtenu à la PRÉPARATION 2 sont mis en solution dans 90 ml de THF, on ajoute 1,89 g du *tert*-butylate de potassium et on laisse une heure à TA. Puis on ajoute goutte à goutte une solution de 4,75 g de bromure de 3,5-bis(trifluorométhyl)benzyle dans 30 ml de THF et laisse une nuit sous agitation à TA. On évapore, reprend par de l'eau, extrait par AcOEt puis on lave 2 fois à l'eau puis par une solution saturée de chlorure de sodium, sèche sur $MgSO_4$ et évapore sous vide le solvant. On obtient 8,4 g du produit attendu.

B) 5-(3,4-Difluorophényl)-5-(2-hydroxyéthyl)-1-[3,5-bis(trifluorométhyl) benzyl] pipérid-2-one.

On met en solution dans 70 ml de méthanol le produit obtenu à l'étape précédente (8,4 g) et l'on ajoute une solution d'éther chlorhydrique jusqu'à obtenir un pH inférieur à 1. Après 15 minutes, on évapore, reprend le résidu par une solution saturée d'HCl gaz dans le MeOH et concentre sous vide le solvant. On reprend par DCM et lave par une solution à 5 % de $NaHCO_3$ puis par une solution saturée de chlorure de sodium, sèche sur $MgSO_4$ et évapore sous vide le solvant. On obtient 5,1 g du produit attendu qui cristallise dans l'éther. F = 120°C.

C) 5-(3,4-Difluorophényl)-5-(2-méthanesulfonyloxyéthyl)-1-[3,5-bis(trifluoro méthyl)benzyl]pipérid-2-one.

On prépare une solution de 4 g du composé obtenu à l'étape précédente dans 50 ml de DCM, on ajoute 1 g du triéthylamine puis on refroidit dans un bain de glace et on ajoute goutte à goutte 1,04 g de chlorure de méthanesulfonyle dans 20 ml de DCM. On évapore, reprend par de l'eau et extrait par AcOEt puis on lave successivement par de l'eau, une solution HCl 2N, de l'eau, une solution $NaHCO_3$ à 5 %, de l'eau, une solution saturée de chlorure de sodium. On sèche sur $MgSO_4$ et évapore sous vide le solvant. On obtient 4,7 g du produit attendu.

D) Chlorure de 1-[2-[1-[3,5-bis(trifluorométhyl)benzyl]-5-(3,4-difluorophényl)2-oxopipérid-5-yl]éthyl]-4-phénylquinuclidinium, dihydrate.

On mélange 0,60 g de 4-phénylquinuclidine et 1,5 g du produit obtenu à l'étape précédente dans le minimum de DMF et on chauffe à 100°C pendant 3 heures. On laisse revenir à TA, évapore, reprend au DCM puis on lave successivement par de l'eau, une solution HCl 2N (3 fois), de l'eau, une solution saturée de chlorure de sodium, sèche sur $MgSO_4$ et évapore sous vide le solvant. On obtient 1 g du produit attendu après trituration dans l'éther, puis essorage. F = 125°C.

EXEMPLE 3

Chlorure de 1-[2-[1-[3,5-bis(trifluorométhyl)brnzyl]-6-(3,4-dichlorophényl)-2-oxoperhydroazepin-6-yl]éthyl]-4-phényl-quinuclidinium, monohydrate.

(I) : Am = 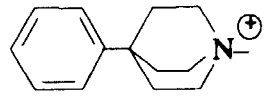 ; Ar = 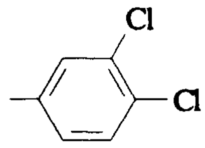 ;

m=2; n=2;

Z = 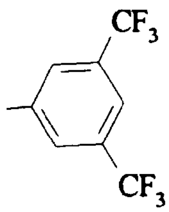 ;

$A^{\ominus} = Cl^{\ominus}$

A) 6-(3,4-Dichlorophényl)-6-[2-(tétrahydropyran-2-yl)éthyl]-1-[3,5-bis(trifluorométhyl)benzyl]perhydroazépin-2-one.

1 g du composé obtenu à la PREPARATION 3 est mis en solution dans 15 ml de THF, on ajoute 0,33 g de *tert*-butylate de potassium et on laisse 1 heure à TA puis on ajoute goutte à goutte 0,71 g de chlorure de 3,5-bis(trifluorométhyl)benzyle dans 5 ml de THF. Après 2 heures, on évapore, reprend par de l'eau et extrait par AcOEt puis on lave par de l'eau et une solution saturée de chlorure de sodium. On sèche sur $MgSO_4$ et évapore sous vide le solvant. On obtient 1,5 g du produit attendu.

B) 6-(3,4-Dichlorophényl)-6-(2-hydroxyéthyl)1- [3,5-bis(trifluoro méthyl)benzyl]perhydroazépin-2-one.

1,5 g du produit obtenu à l'étape précédente sont mis en solution dans 20 ml de méthanol, on ajoute de l'éther chlorhydrique jusqu'à atteindre un pH inférieur à 1. Après 15 minutes, on évapore, reprend 2 fois par du méthanol chlorhydrique et évapore à sec pour obtenir 1,4 g du produit attendu.

C) 6-(3,4-Dichlorophényl)-6-(2-méthanesulfonyloxyéthyl)-1-[3,5-bis(trifluorométhyl)benzyl]perhydroazépin-2-one.

On met en solution dans 15 ml de DCM, 1,4 g du produit obtenu à l'étape précédente, on ajoute 0,32 g de triéthylamine et on refroidit le milieu par un bain de glace. On ajoute goutte à goutte 0,33 g de chlorure de méthanesulfonyle dans 5 ml de DCM et on laisse revenir à TA. On évapore, reprend par de l'eau et extrait par AcOEt puis on lave à l'eau et par une solution saturée de chlorure de sodium. On sèche sur $MgSO_4$, évapore le solvant, puis on chromatographie sur silice en éluant par DCM puis un mélange DCM/MeOH (99/1 ; v/v). On obtient 1,3 g du produit attendu.

D) Chlorure de 1-[2-[1-[3,5-bis(trifluorométhyl)benzyl]-6-(3,4-dichlorophényl)-2-oxoperhydroazepin-6-yl]éthyl]-4-phénylquinuclidinium, monohydrate.

On mélange 0,48 g de 4-phénylquinuclidine et 1,3 g du produit obtenu à l'étape précédente dans un minimum de DMF et on chauffe à 100°C pendant 5 heures. Puis on ajoute 0,63 g d'iodure de sodium et on maintient le chauffage pendant 1 heure. On laisse revenir à TA, évapore le solvant puis reprend par de l'eau et extrait au DCM. On lave à l'eau, par une solution saturée de chlorure de sodium, sèche sur $MgSO_4$ et évapore le solvant. On reprend par de l'éthanol puis on passe le milieu sur une résine chlorhydrique IRA68. On évapore, reprend au DCM et lave 3 fois par de l'eau, 2 fois par une solution HCl 2N, à nouveau 2 fois par de l'eau puis par une solution saturée en chlorure de sodium. On sèche sur $MgSO_4$ et évapore le solvant. Le produit attendu est trituré dans l'éther puis essoré; on obtient 0,8 g, F = 157°C.

En procédant comme à l'EXEMPLE 1, on a également préparé les composés décrits dans le tableau 1 ci-dessous.

TABLEAU 1

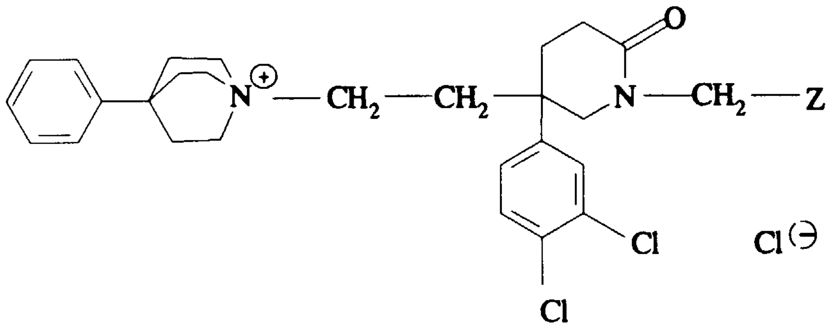

(I)

| EXEMPLE n° | Z | Solvate ; F°C |
|---|---|---|
| 4 | 3,5-diméthylphényle | $2H_2O$ 154-156 |
| 5 | 2,4-bis(trifluorométhyl)phényle | $2{,}5H_2O$ 148-150 |
| 6 | 3-trifluorométhylphényle | $2H_2O$ 164-166 |

EXEMPLES 7 et 8

Iodure de 1-méthyl-1-[2-[1-[3,5-bis(trifluorométhyl)benzyl]-5-(3,4-dichlorophényl)-2-oxopipérid-5-yl]éthyl]-4-phénylpipéridinium (isomère axial et isomère équatorial).

EXEMPLE 7 (I) :

Am = 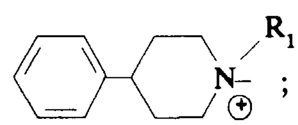 ;

$R_1$ = Me en position axiale ;

Ar = 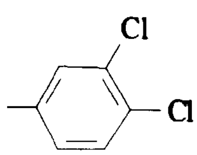 ;

n = 1 ;

Z = 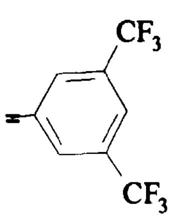 ;

$A^{\ominus} = I^{\ominus}$ ; m = 2

EXEMPLE 8 (I) :

Am = 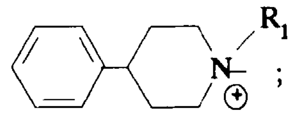 ;

$R_1$ = Me en position équatoriale ;

Ar = 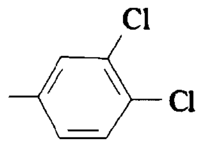 ;

n = 1 ;

Z = 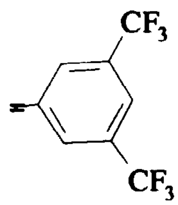 ;

A⊖ = I⊖ ; m = 2

A) Chlorhydrate de 1-[2-[1-[3,5-bis(trifluorométhyl)benzyl]-5-(3,4-dichlorophényl)-2-oxopipérid-5-yl]éthyl]-4-phénylpipéridine.

On mélange 2,79 g de 5-(2-mésyloxyéthyl)-5-(3,4-dichlorophényl)-1-[3,5-bis(trifluorométhyl)benzyl]pipérid-2-one préparé à l'exemple 1, étape B, 1,95 g de $K_2CO_3$ et 0,91 g de 4-phénylpipéridine dans 6 ml d'acétonitrile et 6 ml de DMF et on chauffe à 80°C pendant 4 heures. Après évaporation des solvants, on extrait à l'acétate d'éthyle, lave à l'eau puis par une solution saturée de NaCl. On sèche sur $MgSO_4$, concentre puis on chromatographie sur silice en éluant par DCM/MeOH (100/1 ; v/v). On reprend un échantillon du produit obtenu dans une solution d'éther chlorhydrique et évapore le solvant. On obtient 0,365 g du produit attendu qui cristallise dans le mélange pentane/éther, F = 170°C

B) Iodure de 1-méthyl-1-[2-[1-[3,5-bis(trifluorométhyl)benzyl]-5-(3,4-dichloro phényl)-2-oxopipérid-5-yl]éthyl]-4-phénylpipéridinium (isomère axial et isomère équatorial).

On laisse sous agitation pendant 18 heures à TA un mélange comprenant 1,1 g du composé préparé à l'étape précédente sous forme de base dans 10 ml d'iodure de méthyle. On évapore l'iodure de méthyle en excès, reprend à l'éther, filtre puis on reprend par du DCM et évapore. Le résidu est chromatographié sur silice. En éluant par un mélange DCM-MeOH (100/3 ; v/v), on obtient le composé le moins polaire dans lequel le méthyle est en position axiale sur l'azote de la pipéridine, F = 128°C. En éluant par un mélange DCM-MeOH (100/15 ; v/v), on obtient le composé le plus polaire sur lequel le méthyle est en position équatoriale sur l'azote de la pipéridine, F =166°C.

EXEMPLE 9

Chlorure de 1-[2-[1-[3,5-bis(trifluorométhyl)benzyl]-4-(3,4-dichlorophényl)-2-oxopyrrolidin-4-yl]éthyl]-4-phénylquinuclidinium, dihydrate.

(I) : Am = 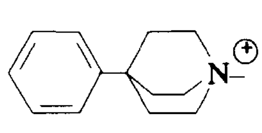 ; Ar = 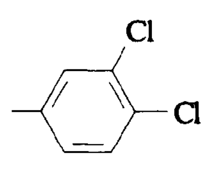 ;

m=2; n = 0 ;

Z = 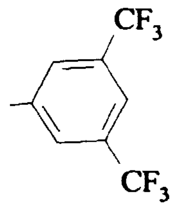 ;

$A^{\ominus} = Cl^{\ominus}$

A) 4-(3,4-Dichlorophényl)-1-[3,5-bis(trifluorométhyl)benzyl]-4-[2-(tétrahydropyran-2-yloxy)éthyl] pyrrolidin-2-one.

On agite pendant 1 heure à TA un mélange contenant 3 g du composé obtenu à la Préparation 4 et 0,959 g de *tert*-butylate de potassium dans 80 ml de THF, puis on ajoute 2,24 g de chlorure de 3,5-bis(trifluorométhyl)benzyle et on chauffe à 50°C pendant 3 heures. On refroidit à TA, évapore les solvants puis on extrait par AcOEt, lave à l'eau, par une solution saturée de chlorure de sodium. La phase organique est séchée sur $MgSO_4$ puis concentrée et le résidu est chromatographié sur silice en éluant par DCM/MeOH (100/1 ; v/v). On obtient 2,3 g du produit attendu.

B) 4-(3,4-Dichlorophényl)-1-[3,5-bis(trifluorométhyl)benzyl]-4-[2-(mésyloxy) éthyl]pyrrolidin-2-one.

On dissout 2,3 g du composé obtenu à l'étape précédente dans 100 ml de méthanol et l'on ajoute de l'éther chlorhydrique jusqu'à pH inférieur à 1. On agite 15 minutes, évapore, reprend par AcOEt, lave à l'eau puis par une solution saturée de chlorure de sodium. On sèche sur $Na_2SO_4$ et évapore. 2 g de l'alcool ainsi obtenu sont placés dans 50 ml de DCM en présence de 0,61 g de triéthylamine et l'on ajoute goutte à goutte à froid une solution contenant 0,51 g de chlorure de méthanesulfonyle et 10 ml de DCM puis on agite à TA pendant 15 minutes. On évapore le milieu réactionnel, extrait à l'AcOEt, lave à l'eau, puis par une solution saturée de chlorure de sodium. Aprés séchage sur $Na_2SO_4$ et évaporation du solvant, on obtient 2 g du produit attendu.

C) Chlorure de 1-[2-[1-[3,5-bis(trifluorométhyl)benzyl]-4-(3,4-dichlorophényl)-2-oxopyrrolidin-4-yl]éthyl]-4-phénylquinuclidinium, dihydrate.

On chauffe à 80°C pendant 4 heures un mélange contenant 1,12 g du composé obtenu à l'étape précédente et 0,544 g de 4-phénylquinuclidine dans 2 ml de DMF. On refroidit à TA puis on ajoute de la glace et de l'eau. Le milieu réactionnel est extrait au DCM puis on lave 6 fois par HCl 2N, puis par de l'eau et par une solution saturée de chlorure de sodium. On sèche sur $Na_2SO_4$ et évapore sous vide le solvant. On obtient 0,78 g du produit attendu, F = 120-122°C.

EXEMPLE 10

Chlorhydrate de 4-(3,4-dichlorophényl)-4-[2-(4-hydroxy-4-phénylpipérid-1-yl) éthyl]-1-[3,5-bis(trifluorométhyl)benzyl] pyrrolidin-2-one.

(I) : Am = 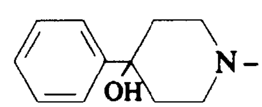 ; Ar = 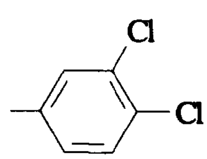

n = 0 ;

Z = 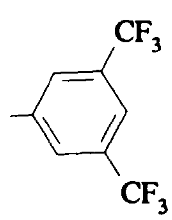 ;

m = 2

Dans 2 ml de DMF on prépare un mélange contenant 1 g de 4-(3,4-dichlorophényl)-1-[3,5-bis(trifluorométhyl) benzy]-4-[2-(mésyloxy)éthyl] pyrrolidin-2-one obtenu à l'exemple 9, étape B, 0,875 g de 4-hydroxy-4-phénylpipéridine

et 0,483 g de carbonate de potassium et on chauffe à 80°C pendant 3 heures. On laisse refroidir puis on ajoute de la glace, de l'eau et de l'acétate d'éthyle. On extrait par AcOEt, lave à l'eau puis par une solution de chlorure de sodium. Le produit pur est obtenu par chromatographie sur silice en éluant par un mélange DCM-MeOH (100/3 ; v/v). On prépare ensuite le chlorhydrate par action de l'acide chlorhydrique dans DCM. Le produit attendu cristallise dans un mélange pentane-éther, m = 0,555 g, F = 136-138C.

EXEMPLE 11

Chlorhydrate de 4-(3,4-dichlorophényl)-4-[2-(4-phénylpipérid-1-yl)éthyl]-1-[3,5-bis(trifluorométhyl)benzyl]pyrrolidin-2-one, hémihydrate.

(I) : Am = 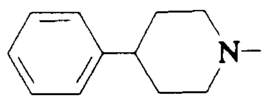 ; Ar = 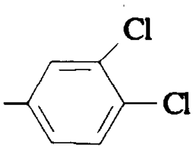

n= 0 ;

Z = 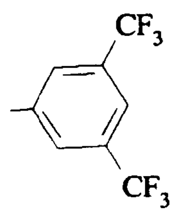 ;

m = 2

On chauffe à 80°C pendant 4 heures un mélange de 2 g du composé obtenu à l'étape B de l'EXEMPLE 9, 0,67 g de 4-phénylpipéridine, 1,43 g de carbonate de potassium dans 6 ml d'acétonitrile et 6 ml de DMF. On concentre sous vide le mélange réactionnel, extrait le résidu à l'AcOEt, lave trois fois la phase organique à l'eau, par une solution saturée de chlorure de sodium, sèche sur $Na_2SO_4$ et évapore sous vide le solvant. On chromatographie le résidu sur silice en éluant par le mélange DCM/MeOH de (100/1; v/v) à (100/3 ; v/v). on dissout le produit obtenu dans du DCM, acidifie à pH = 1 par ajout d'une solution saturée d'HCl gaz dans l'éther et concentre sous vide. On obtient 0,39 g du produit attendu après cristallisation dans le pentane, F = 126-130°C.

EXEMPLE 12

Chlorure de 1-[3-[1-[3,5-bis(trifluorométhyl)bcnzyl]-5-(3,4-dichlorophényl)-2-oxopipérid-5-yl]propyl]-4-phénylquinuclidinium.

(I) : Am = 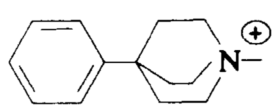 ; Ar = 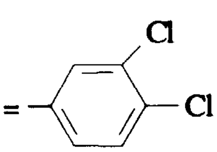 ;

m = 3 ; n = 1 ;

Z = 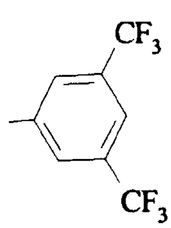 ;

$A^{\ominus} = Cl^{\ominus}$

A) 5-(3,4-Dichlorophényl)-5-[3-(tétrahydropyran-2-yloxy)propyl]-1-[3,5-bis(trifluorométhyl)benzyl]pipérid-2-one.

A une solution de 6,8 g du composé obtenu à la PREPARATION 5 dans 100 ml de THF, on ajoute 1,98 g de *tert*-butylate de potassium et laisse 1 heure sous agitation à TA. Puis on ajoute 4,62 g de chlorure de 3,5-bis(trifluorométhyl) benzyle et chauffe à 50'C pendant 2 heures le mélange réactionnel. On concentre sous vide, extrait le résidu à l'AcOEt, lave la phase organique à l'eau, par une solution saturée de NaCl, sèche sur $MgSO_4$ et évapore sous vide le solvant. On chromatographie le résidu sur silice en éluant par le mélange DCM/MeOH (100/1 ; v/v). On obtient 10 g du produit attendu.

B) 5-(3,4-Dichlorophényl)5-(3-hydroxypropyl)-1-[3,5-bis(trifluorométhyl) benzyl]pipérid-2-one.

A une solution de 10 g du composé obtenu à l'étape précédente dans 150 ml de MeOH, on ajoute jusqu'à pH < 1 une solution saturée d'HCl gaz dans l'éther et laisse 10 minutes sous agitation à TA. On concentre sous vide, extrait le résidu au DCM, lave la phase organique à l'eau, par une solution à 5 % de $NaHCO_3$, par une solution saturée de NaCl, sèche sur $MgSO_4$ et évapore sous vide le solvant. On obtient 8,8 g du produit attendu sous forme d'huile.

C) 5-(3,4-Dichlorophényl)-5-[3-(méthanesulfonyloxy)propyl]-1-[3,5-bis(trifluorométhyl)benzyl]pipérid-2-one.

On refroidit au bain de glace une solution de 8,8 g du composé obtenu à l'étape précédente et 2,51 g de triéthylamine dans 100 ml de DCM, ajoute goutte à goutte une solution de 2,09 g de chlorure de méthanesulfonyle dans 20 ml de DCM et laisse 10 minutes sous agitation. On concentre sous vide, extrait le résidu à l'AcOEt, lave la phase organique à l'eau, par une solution saturée de NaCl, sèche sur $MgSO_4$ et évapore sous vide le solvant. On obtient 8,5 g du produit attendu.

D) Chlorure de 1-[3-[1-[3,5-bis(trifluorométhyl)benzyl]-5-(3,4-dichlorophényl)-2-oxopipérid-5-yl]propyl]-4-phénylquinuclidinium.

On chauffe à 80°C pendant 4 heures un mélange de 2,41 g du composé obtenu à l'étape précédente et 0,996 g de 4-phénylquinuclidine dans 4 ml de DMF. Après refroidissement à TA, on verse le mélange réactionnel dans un mélange eau/glace, extrait au DCM, lave la phase organique à l'eau, quatre fois par une solution d'HCl 2N, trois fois par une solution saturée de NaCl, sèche sur $MgSO_4$ et évapore sous vide le solvant. On obtient 1,45 g du produit attendu après trituration dans le mélange pentane/éther puis essorage, F = 132-134°C.

## Revendications

**1.** Un composé de formule :

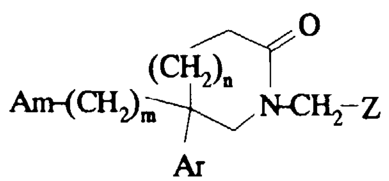

(I)

dans laquelle :

- m est deux ou trois ;
- n est 0, 1 ou 2;
- Am représente un groupe choisi parmi :

a)

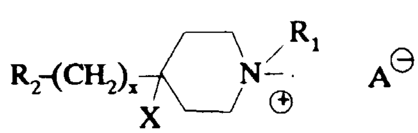

b)

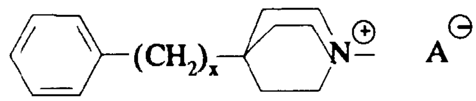

et lorsque n = 0, Am peut également représenter le groupe :
c)

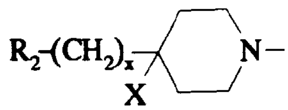

- $R_1$ represente un ($C_1$-$C_4$)alkyle ou un benzyle ;
- $R_2$ représente un phényle non substitué ou substitué une ou plusieurs fois par un atome d'halogène, un hydroxyle, un ($C_1$-$C_4$)alcoxy, un ($C_1$-$C_4$)alkyle, un trifluorométhyle, lesdits substituants étant identiques ou différents ;
- X représente un atome d'hydrogène ou un groupe hydroxyle ;
- x est zéro ou un ;
- Ar représente un phényle non substitué ou substitué une ou plusieurs fois par un atome d'halogène ; un naphtyle ou un indolyle ;
- Z représente un phényle substitué une ou plusieurs fois par un atome d'halogène, un ($C_1$ -$C_4$) alkyle, un trifluorométhyle;
- $A^{\ominus}$ représente un anion ;

et ses sels éventuels avec des acides minéraux ou organiques et ses solvates éventuels.

**2.** Un composé de formule (I) selon la revendication 1 dans laquelle m = 2 ;

**3.** Un composé de formule (I) selon la revendication 1 dans laquelle Z représente un groupe choisi parmi diméthylphényle, trifluorométhylphényle et bistrifluorométhylphényle.

**4.** Un composé selon la revendication 1 de formule (I) dans laquelle :

- Ar représente un 3,4-dichlorophényle ou un 3,4-difluorophényle ;
- Z représente un 3,5-diméthylphényle, un 3,5-bis(trifluorométhyl)phényle ou un 2,4-bis(trifluorométhyl)phényle; et
- soit n = 0, 1 ou 2 et Am représente un groupe a) ou b) tel que défini dans la revendication 1, soit n = 0 et Am représente un groupe c) tel que défini dans la revendication 1;
- $A^{\ominus}$ représente un anion pharmaceutiquement acceptable.

**5.** Un composé selon la revendication 1 de formule :

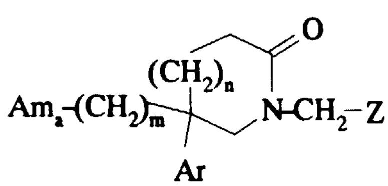

(Ia)

dans laquelle $Am_a$ représente le groupe b) tel que défini pour (I) dans la revendication 1 et n, m, Ar et Z sont tels que définis pour (I) dans la revendication 1
et ses solvates éventuels.

**6.** Un sel pharmaceutiquement acceptable d'un composé de formule :

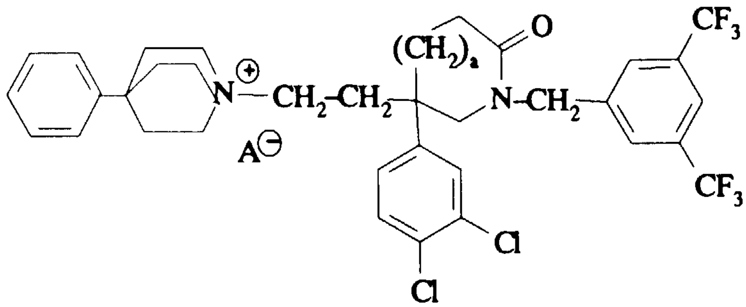 (I')

dans laquelle a est 0 ou 1 et $A^{\ominus}$ est un anion pharmaceutiquement acceptable et ses solvates éventuels.

**7.** Un procédé pour la préparation des composés de formule (I) selon la revendication 1 caractérisé en ce que :

1) on traite un composé de formule :

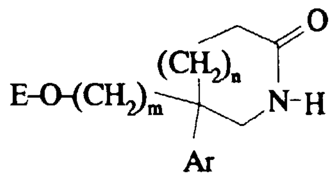 (II)

dans laquelle m, n et Ar sont tels que définis pour (I) dans la revendication 1 et E représente un groupe O-protecteur tel que le tétrahydropyran-2-yle, le benzoyle ou un acyle en $C_1$-$C_4$ ;
avec un dérivé halogéné de formule :

$$Hal\text{-}CH_2\text{-}Z \qquad (III)$$

dans laquelle Z est tel que défini pour (I) dans la revendication 1 et Hal représente un atome d'halogène, préférentiellement le brome ou le chlore ;
2) on transforme le groupe O-protecteur en un groupe hydroxyle par action d'un acide ou d'une base ;
3) on traite l'alcool ainsi obtenu de formule :

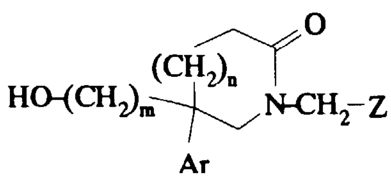 (IV)

dans laquelle m, Ar, Z et n sont tels que définis pour (I) dans le revendication 1 ;
avec le chlorure de méthanesulfonyle, le chlorure de benzènesulfonyle, le chlorure de paratoluènesulfonyle ou le chlorure de trifluorométhanesulfonyle ; 4) on fait réagir le composé ainsi obtenu de formule :

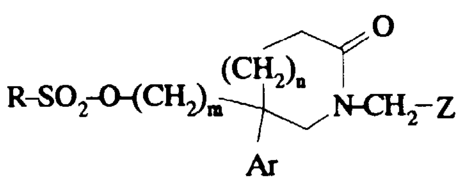 (V)

dans laquelle R représente un groupe méthyle, phényle, paratolyle ou trifluorométhyle ;
avec une amine secondaire ou tertiaire AmH ou Am ;
5) on isole le produit ainsi obtenu et, éventuellement, on échange l'anion sulfonate du sel quaternaire avec un autre anion et, éventuellement, on transforme le produit obtenu en un des ses sels.

**8.** Composition pharmaceutique contenant à titre de principe actif un composé selon l'une quelconque des revendications 1 à 6 ou un de leurs sels pharmaceutiquement acceptables.

**9.** Composition pharmaceutique selon la revendication 8, sous forme d'unité de dosage, dans laquelle le principe actif est mélangé à au moins un excipient pharmaceutique.

**10.** Composition pharmaceutique selon la revendication 9, contenant de 0,5 à 1000 mg de principe actif.

Office européen des brevets

# RAPPORT DE RECHERCHE EUROPEENNE

Numero de la demande
EP 96 40 0202

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X,D | EP-A-0 512 901 (ELF SANOFI) 11 Novembre 1992<br>* revendication 1; exemples *<br>--- | 1-10 | C07D211/76<br>C07D453/02<br>A61K31/445 |
| D,A | EP-A-0 591 040 (ELF SANOFI) 6 Avril 1994<br>----- | 1-10 | |
| | | | **DOMAINES TECHNIQUES RECHERCHES (Int.Cl.6)** |
| | | | C07D |

Le présent rapport a été établi pour toutes les revendications

| Lieu de la recherche | Date d'achèvement de la recherche | Examinateur |
|---|---|---|
| LA HAYE | 20 Mai 1996 | De Jong, B |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)